# EUROPEAN PATENT APPLICATION

(11) **EP 4 685 487 A1**
(43) Date of publication of application: **28.01.2026**
(21) Application number: 24774988.0
(22) Date of filing: 22.03.2024
(51) Int. Cl.: G01N 33/53, C07K 16/18, C07K 17/00, C12M 1/34, C12N 5/20, G01N 33/543, G01N 33/545

(54) **METHOD FOR MEASURING PULMONARY SURFACTANT PROTEIN D, MEASUREMENT KIT, MONOCLONAL ANTIBODY, AND CELL**

(30) Priority: 23.03.2023 JP 2023046633
(71) Applicant: Sekisui Medical Co., Ltd., Tokyo 103-0027 (JP)
(72) Inventor: HIROTA, Jiro, Tokyo 103-0027 (JP); SHIMIZU, Tomo, Tokyo 103-0027 (JP); KOUHATA, Tomohiro, Tokyo 103-0027 (JP); IWASAKI, Manami, Tokyo 103-0027 (JP)
(74) Representative: Ter Meer Steinmeister & Partner
(86) International application number: PCT/JP2024/011227
(87) International publication number: WO 2024/195854

(57) **Abstract**

This method for measuring pulmonary surfactant protein D includes a step of bringing a sample containing pulmonary surfactant protein D into contact with an insoluble carrier on which an anti-pulmonary surfactant protein D monoclonal antibody has been immobilized, and a step of detecting a complex of the pulmonary surfactant protein D and at least two of the anti-pulmonary surfactant protein D monoclonal antibodies, wherein the insoluble carrier supports only one type of antibody as the anti-pulmonary surfactant protein D monoclonal antibody.

## Description

### [Technical Field]

The present invention relates to a method for measuring pulmonary surfactant protein D, a measurement kit, a monoclonal antibody, and a cell.

Priority is claimed on Japanese Patent Application No. 2023-046633, filed March 23, 2023, the content of which is incorporated herein by reference.

### [Background Art]

Pulmonary surfactants are proteins that are produced and secreted by type II alveolar epithelial cells in the lungs. Pulmonary surfactants have the function of facilitating swelling of the pulmonary alveoli and assisting respiration and gas exchange by weakening the surface tension at the surfaces of the alveoli.

The pulmonary surfactants include pulmonary surfactant protein A (hereinafter also abbreviated as SP-A), pulmonary surfactant protein B (hereinafter also abbreviated as SP-B), pulmonary surfactant protein C (hereinafter also abbreviated as SP-C), and pulmonary surfactant protein D (hereinafter also abbreviated as SP-D). SP-A and SP-D are hydrophilic glycoproteins. SP-A and SP-D exhibit biological defense actions, and bind with specific lipids. SP-B and SP-C are hydrophobic proteins, and form assemblies with phospholipids.

Measurement of SP-D is useful in diagnosing interstitial pneumonia, as an activity indicator, and in prognosis prediction. Further, because SP-D decreases rapidly when a treatment of interstitial pneumonia is effective, SP-D is also used to observe treatment progress. Measurement of SP-D in the blood can be conducted, for example, by sandwich ELISA (Enzyme-Linked Immunosorbent Assay) using two types of antibodies, as disclosed in Non-Patent Document 1.

### [Citation List]

### [Non-Patent Document]

[Non-Patent Document 1]
Preston E. Bratcher et al., Factors Influencing the Measurement of Plasma/Serum Surfactant Protein D Levels by ELISA PLOS ONE, November 2014, Volume 9, Issue 11.

### [Summary of Invention]

### [Technical Problem]

SP-D is known to form a wide variety of multimers from trimers to 36-mers or even higher order multimers in vivo. The distribution of SP-D multimers in the blood differs depending on the physical condition and pathology of the sample provider. On the other hand, in conventional SP-D measurement methods such as sandwich ELISA, changes in the number of monomers constituting a multimer (for example, a change from a dodecamer to a 36-mer) have sometimes be accompanied by epitope concentration causing fluctuations in avidity, or changes in the exposed surfaces of epitopes, resulting in variation in the reactivity between the antibodies and the various SP-D multimers. Accordingly, differences in the distribution of SP-D multimers can sometimes result in changes in the SP-D quantitative value.

Objects of the present invention are to provide a measurement method and measurement kit for pulmonary surfactant protein D that enable accurate quantification regardless of the number of monomers constituting the pulmonary surfactant protein D multimer, and also to provide a monoclonal antibody that can be used in this measurement method and measurement kit, and a hybridoma that produces this antibody.

### [Solution to Problem]

The present invention includes the following aspects.
[1] A method for measuring pulmonary surfactant protein D, the method including:
   a step of bringing a sample containing pulmonary surfactant protein D into contact with an insoluble carrier on which an anti-pulmonary surfactant protein D monoclonal antibody has been immobilized, and
   a step of detecting a complex of the pulmonary surfactant protein D and at least two of the anti-pulmonary surfactant protein D monoclonal antibodies, wherein
   only one type of antibody is immobilized on the insoluble carrier as the anti-pulmonary surfactant protein D monoclonal antibody.
[2] The method according to [1], wherein the insoluble carrier is composed of latex particles.
[3] The method according to [1], wherein
   the insoluble carrier has a plate-like form, and
   the complex contains the anti-pulmonary surfactant protein D monoclonal antibody immobilized on the insoluble carrier, and a labeled antibody containing the anti-pulmonary surfactant protein D monoclonal antibody and a labeling material.
[4] The method according to any one of [1] to [3], wherein the sample is blood serum or blood plasma.
[5] The method according to any one of [1] to [4], wherein
   the pulmonary surfactant protein D includes at least one basic unit composed of a trimer of pulmonary surfactant protein D, and
   only one anti-pulmonary surfactant protein D monoclonal antibody binds to a single basic unit.
[6] The method according to any one of [1] to [5], wherein
   in the step of detecting the complex, trimers of pulmonary surfactant protein D are not detected.
[7] The method according to any one of [1] to [6], wherein
   in the step of detecting the complex, pulmonary surfactant protein D having two or more assembled basic units is detected.
[8] A measurement kit for pulmonary surfactant protein D including:
   a first reagent containing an insoluble carrier on which an anti-pulmonary surfactant protein D monoclonal antibody has been immobilized, and
   a second reagent that contains a labeled antibody containing the anti-pulmonary surfactant protein D monoclonal antibody and a labeling material.
[9] The measurement kit according to [8], wherein the insoluble carrier and the labeling material are the same substance.
[10] A monoclonal antibody that binds to a trimer of pulmonary surfactant protein D, wherein only one antibody binds to a single trimer of pulmonary surfactant protein D.
[11] The monoclonal antibody according to [10], wherein at least two antibodies bind to a dodecamer of pulmonary surfactant protein D.
[12] A hybridoma that produces the monoclonal antibody according to [10] or [11].
[13] A hybridoma deposited with National Institute of Technology and Evaluation Patent Microorganisms Depositary with an accession number NITE BP-03825 or an accession number NITE BP-03826.

### [Advantageous Effects of Invention]

The aspects described above are able to provide a measurement method and measurement kit for pulmonary surfactant protein D that enable accurate quantification regardless of the number of monomers constituting the pulmonary surfactant protein D multimer, as well as providing a monoclonal antibody that can be used in this measurement method and measurement kit, and a cell that produces this antibody.

### [Brief Description of the Drawings]

FIG. 1 is a graph illustrating the reactivity between anti-SP-D monoclonal antibodies and biological sample-derived SP-D.
FIG. 2 is a chromatogram illustrating the results of analyzing rSP-D manufactured by GenScript Biotech Corporation by gel filtration chromatography.
FIG. 3 is a chromatogram illustrating the results of analyzing rSP-D manufactured by R&D Systems, Inc., and rSP-D manufactured by GenScript Biotech Corporation using gel filtration chromatography.
FIG. 4 is a graph illustrating the measured value for the SP-D concentration in each of the fractions obtained in analysis 5 in Example 2.
FIG. 5 is a graph illustrating the measured value for the SP-D concentration in each of the fractions obtained in analysis 5 in Comparative Example 3.
FIG. 6 is a graph illustrating the measured value for the SP-D concentration in each of the fractions obtained in analysis 5 in Comparative Example 4.

### [Description of Embodiments]

### <Method for Measuring Pulmonary Surfactant Protein D>

A method for measuring pulmonary surfactant protein D (hereinafter abbreviated as SP-D) according to one aspect of the present invention includes a step of bringing a sample containing SP-D into contact with an insoluble carrier on which an anti-SP-D monoclonal antibody has been immobilized, and a step of detecting a complex of the SP-D and at least two anti-SP-D monoclonal antibodies, wherein the insoluble carrier supports only one type of antibody as the anti-SP-D monoclonal antibody.

The method for measuring SP-D according to this aspect of the present invention is described below in further detail. However, the embodiments described below are merely examples of implementing the invention, and do not preclude various modifications or technical applications not mentioned in the embodiments. In other words, the embodiments may be implemented with any of various modifications without departing from the scope of the present invention.

### (Samples)

In this description, a "sample" refers mainly to a bodily fluid derived from a living organism. Examples of these samples include blood, blood serum, blood plasma, and amniotic fluid, although blood serum and blood plasma are preferred. Examples of the subjects from which samples may be extracted include humans and animals (for example, monkeys, dogs and cats), but humans are preferred. The sample may be an unmodified sample extracted from the subject, or may be a treated sample in which the extracted sample has been subjected to a typical treatment such as dilution or concentration. The people performing the extraction and preparation of the samples used in the present invention may be the same people who perform the measurement method of the present invention, or may be different people. Further, the samples used in the measurement method of the present invention may be extracted or prepared at the time of implementing the measurement method of the present invention, or may be stored samples that have been extracted or prepared in advance.

### (SP-D)

SP-D is a monomer, but forms a trimer composed of three assembled monomers. In this description, this trimer is sometimes referred to as a basic unit. SP-D also forms a dodecamer composed of an assembly of four of these basic units. Moreover, this dodecamer undergoes further assembly to form a 36-mer and the like. In this description, an SP-D multimer refers to a structure formed by assembling two or more basic units. The SP-D multimer is preferably a structure formed by assembling four or more basic units. In this description, the abbreviation "SP-D" is a generic term that includes the monomer, the trimer and the various multimers of SP-D.

### (Monoclonal Antibodies)

In this description, a "monoclonal antibody" means an antibody or antibody molecule obtained from a hybridoma derived from a single antibody-producing cell. In the measurement method of the present invention, provided the effects of the present invention can be achieved, an antibody fragment having the same function as this monoclonal antibody may also be used. Examples of antibody fragments having the same function as the monoclonal antibody include functional fragments containing the Fab portion of the monoclonal antibody obtained by enzymatic digestion of the monoclonal antibody, functional fragments containing the Fab portion of the monoclonal antibody produced by gene recombination, and functional fragments containing scFv produced by the phage display method.

### (Method for Preparing Monoclonal Antibody)

The monoclonal antibody used in one aspect of the present invention can be prepared by using, as an antigen (also referred to as an immunogen), a solution prepared by dissolving SP-D in a solvent such as phosphate buffered saline, and then immunizing a non-human animal with this solution. If necessary, a suitable adjuvant may be added to the solution, and an emulsion then used to perform the immunization. Examples of adjuvants that may be used include typically employed adjuvants such as water-in-oil emulsifiers, water-in-oil-in-water emulsifiers, oil-in-water emulsifiers, liposomes and aluminum hydroxide gel, as well as proteins or peptidic substances derived from biological components. For example, incomplete Freund's adjuvant or complete Freund's adjuvant or the like can be used favorably. There are no particular limitations on the administration pathway, administration dose and administration period for the adjuvant, which are preferably selected appropriately so as to achieve the desired enhanced immune response in the animal being immunized with the antigen.

There are no particular limitations on the type of animal used in the immunization, and examples of animals that may be used favorably include mammals such as mice, rats, cows, rabbits, goats, sheep, alpacas, mice and rats with the use of mice or rats being more desirable. Immunization of the animals may be conducted in accordance with typical techniques, and for example, may be conducted by injecting a solution of the antigen, and preferably a mixture containing an adjuvant, subcutaneously, intradermally, intravenously or intraperitoneally. The immune response typically varies depending on the variety and strain of animal being immunized, and therefore the immunization schedule is preferably determined appropriately in accordance with the animal being used. Administration of the antigen is preferably repeated several times following the initial immunization.

The subsequent operations described below may be used to obtain the monoclonal antibody of the present invention, but the invention is not limited to these particular operations. Production methods for monoclonal antibodies themselves are well known in the technical field, and in widespread use. Accordingly, a person skilled in the art can readily prepare the monoclonal antibody of the present invention by using the type of antigen described above (for example, by reference to Antibodies, Laboratory Manual (Cold Spring Harbor Laboratory Press (1988), chapter 6 or the like).

Following the final immunization, a hybridoma can be obtained by extracting the spleen cells or lymph node cells that represent the antibody-producing cells from the immunized animal, and performing cell fusion with a myeloma-derived cell line having a high proliferative potential. A cell having superior antibody-producing potential (qualitatively and quantitatively) is preferably used in the cell fusion. Further, the myeloma-derived cell line preferably has good compatibility with the animal that yields the antibody-producing cells to be fused. The cell fusion can be conducted using conventional techniques widely known in the field. For example, the polyethylene glycol method, a method using the Sendai virus, or a method that utilizes an electric current may be employed. The obtained hybridoma can be proliferated under conditions commonly employed in the field. The desired hybridoma can be selected while confirming the properties of the produced antibody. Cloning of the hybridoma can be conducted, for example, using known methods such as the limiting dilution method or the soft agar method.

After the cloning step, the binding ability between the produced monoclonal antibody and SP-D can be assayed using a method such as the ELISA method, the RIA method, or a fluorescent antibody method. These operations can be used to confirm whether or not the selected hybridoma produces a monoclonal antibody having the desired properties.

A monoclonal antibody having the desired characteristics can be produced by mass cultivation of the hybridoma selected in the manner described above. Although there are no particular limitations on the mass cultivation method, suitable methods include a method in which the monoclonal antibody is produced in a culture medium by cultivating the hybridoma in an appropriate culture medium, and a method in which the hybridoma is injected intraperitoneally into a mammal for proliferation, with the monoclonal antibody being produced in the ascitic fluid.

The anti-SP-D monoclonal antibody of the present invention is preferably an antibody derived from cells obtained using recombinant human SP-D manufactured by GenScript Biotech Corporation as the immunogen, and conducting subcutaneous immunization and intraperitoneal immunization of Balb/c mice or F344/Jcl rats. For example, the anti-SP-D monoclonal antibody is preferably S21208 or S21202 produced from the hybridomas registered with the National Institute of Technology and Evaluation Patent Microorganisms Depositary (Room 122, 2-5-8 Kazusakamatari, Kisarazu-shi, Chiba, Japan) on February 14, 2023 with the accession numbers NITE BP-03826 and NITE BP-03825 respectively.

In the method for measuring SP-D in the present invention, the monoclonal antibody that binds to the SP-D is of only one type, in other words, is composed of only anti-SP-D monoclonal antibodies having the same amino acid sequence, and produces a sandwich system. In this description, the expression "sandwich system" describes a method in which the substance being detected, namely the SP-D, is sandwiched between at least two monoclonal antibodies.

In those cases where a sandwich system is formed, it is preferable that at least one of the two or more anti-SP-D monoclonal antibodies is an immobilized antibody and at least one antibody is a labelled antibody. In this description, the term "immobilized antibody" means a monoclonal antibody that is immobilized either directly or indirectly on a solid-phase carrier, namely an insoluble carrier. In this description, the term "labelled antibody" means a monoclonal antibody that is labelled directly or indirectly with a labeling material described below and well known to those skilled in the art. In this description, the immobilized antibody and the labelled antibody are anti-SP-D monoclonal antibodies of the same type, namely monoclonal antibodies having the same amino acid sequence.

With the anti-SP-D monoclonal antibody, only one anti-SP-D monoclonal antibody binds to the SP-D trimer (namely, the basic unit). It is thought that this mechanism is due to the epitope being concentrated in one location of the basic unit, so that when the first antibody binds to the basic unit, the second antibody is sterically hindered by the first antibody, and is unable to approach the epitope. Accordingly, the basic unit cannot be sandwiched by two anti-SP-D monoclonal antibodies, meaning a complex cannot be formed. As a result, in the measurement method of the present embodiment of the present invention, the SP-D trimer cannot be detected.

On the other hand, in the case of SP-D containing two or more basic units, for example, in the case of a dodecamer or 36-mer of SP-D, two different anti-SP-D monoclonal antibodies can bind to two different basic units. Accordingly, in the measurement method of the present embodiment, SP-D containing two or more basic units, such as a dodecamer or 36-mer, can be detected.

The immobilized antibody can be produced by physically adsorbing or chemically bonding (via a suitable spacer if required) the monoclonal antibody to an insoluble carrier. Examples of insoluble carriers that may be used include solid phases formed from a polymer substrate such as a polystyrene resin, an inorganic substrate such as glass, or a polysaccharide substrate such as cellulose or agarose. There are no particular limitations on the form of the insoluble carrier, and any appropriate form such as a plate-like form (for example, microplates or membranes), beads or particulate form (for example, latex particles or magnetic particles), or a cylindrical form (for example, a test tube) may be selected.

By using a labeled antibody that can bind directly to the monoclonal antibody used in the method for measuring SP-D according to the present invention, the amount of SP-D can be measured. In the present description, an antibody that can bind to the monoclonal antibody of the present invention and has a labeling material bonded thereto is referred to as a secondary antibody. This secondary antibody may be used to bind the labeling material indirectly to the monoclonal antibody of the present invention.

The intensity of the signal emitted by the labeling material or the turbidity generated by agglutination of the labeling material can be measured to determine the amount of SP-D in a sample. Examples of the labeling material used for preparing the labeled antibody include metal complexes, enzymes, insoluble particles, fluorescent substances, chemiluminescent substances, biotin, avidin, radioisotopes, colloidal gold particles, or colored latex. Examples of the method used for binding the labeling material to the monoclonal antibody include various methods used by those skilled in the art, including the glutaraldehyde method, the maleimide method, the pyridyl disulfide method, or the periodic acid method. In those cases where an enzyme such as horseradish peroxidase (HRP) or alkali phosphatase (ALP) is used as the labeling material, the enzyme activity can be measured by using a specific substrate for the enzyme. For example, when the enzyme is HRP, o-phenylenediamine (OPD) or 3,3',5,5'-tetramethylbenzidine (TMB) can be used, whereas in the case of ALP, p-nitrophenyl phosphate can be used to measure the enzyme activity. In those cases where biotin is used as the labeling material, the monoclonal antibody may be labeled with biotin, and reacted with avidin or streptavidin that has been labeled with an enzyme, a dye, or fluorescent labeling material (preferably HRP).

In this description, the process of physically or chemically immobilizing an antigen or antibody on a solid phase or the resulting state of being immobilized is described using the term "immobilized" or "solid phase immobilized ". Further, the terms "analysis", "detection" or "measurement" include the quantification of SP-D.

The SP-D measurement method in the present invention utilizes the reaction between an antigen and an antibody, and is a method for measuring the SP-D concentration contained within a sample. Examples of the SP-D measurement method in the present invention include enzyme-linked immunosorbent assay (ELISA) methods and latex turbidimetric immunoassay (LTIA) methods, although the present invention is not limited to these methods. The immunoassay method of the present invention is preferably an LTIA method.

The SP-D measurement method of the present invention may be an in vivo or in vitro immunoassay method. Further, a sensitizer may be used to enhance the sensitivity. In the method for measuring SP-D according to the present invention, there are no particular limitations on the order in which the anti-SP-D monoclonal antibody and the sample are added to the measurement system, provided the effects of the present invention can be achieved. In other words, the anti-SP-D monoclonal antibody may be added to the measurement system prior to addition of the sample, at the same time as the sample, or after the addition of the sample.

For each employed measurement method, the procedure and principles of the measurement are described below. The following description merely provides examples of the procedure and principles of each measurement in one embodiment of the present invention, but the scope of the present invention is in no way limited by the following description.

In each of the immunoassay methods described below, specific details such as the method for immobilizing the monoclonal antibody on the solid phase, the method used for binding the monoclonal antibody and the labeling material, and the type of labeling material used include those described above, but any method known to those skilled in the art may be used without any particular restrictions.

### (Latex Turbidimetric Immunoassay Method)

The latex turbidimetric immunoassay method is an immunoassay method that utilizes the agglutination of latex particles, which occurs when an anti-SP-D monoclonal antibody immobilized on the surface of latex particles bind to SP-D. There are no particular limitations on the latex particles, provided they are the type of latex particles typically used in in vitro diagnostics. The concentration of the latex particles during measurement of the agglutination reaction, and the average particle size and the like of the latex particles may be set appropriately in accordance with the sensitivity or performance or the like. In those cases where the latex turbidimetric immunoassay method is used as the method for measuring SP-D in the present invention, the measurement procedure and principles are as described below.
(1) Latex particles with the anti-SP-D monoclonal antibody immobilized thereon are brought into contact with the sample.
(2) The SP-D in the sample forms a complex with at least two anti-SP-D monoclonal antibodies, causing the latex particles to agglutinate.
(3) Near infrared light (for example, with a wavelength of 600 nm) is irradiated onto the sample, and a light absorbance measurement or scattered light measurement is conducted. Based on the measured value, the SP-D concentration is determined.

In those cases where the latex turbidimetric immunoassay method is used as the method for measuring SP-D in the present invention, the latex particles function as an insoluble carrier and a labeling material. The monoclonal antibody immobilized on the latex particles that binds to the SP-D is a single type of antibody, namely includes only anti-SP-D monoclonal antibodies having the same amino acid sequence.

Examples of measurement devices that may be used include biochemical autoanalyzers (such as the Hitachi Automatic Analyzer 3500, manufactured by Hitachi, Ltd.).

### (ELISA)

In this description, the abbreviation "ELISA" refers to a method in which the SP-D that represents the detection target substance contained in a sample is captured using an anti-SP-D monoclonal antibody, and is then detected using an enzyme reaction. The solid phase is preferably a plate (also termed an immunoplate). HRP or ALP can be used as the labeling material. In those cases where the sandwich ELISA method is used as the method for measuring SP-D in the present invention, the measurement procedure and principles are as described below.
(1) The sample is brought into contact with a solid phase containing the immobilized anti-SP-D monoclonal antibody, and the SP-D within the sample binds to the anti-SP-D monoclonal antibody, namely to the immobilized antibody.
(2) When the labeled anti-SP-D monoclonal antibody, namely the labeled antibody, is added to and reacted with the solid phase, the labeled antibody binds to the SP-D, forming an antibody-SP-D-labeled antibody complex, namely a sandwich.
(3) Following washing, color development of the labeling material is induced, and the absorbance is measured.

The amount of SP-D in the sample can be measured based on the amount of measured labeling material.

In the sandwich ELISA method, a secondary antibody may also be used. By using a secondary antibody, the reaction can be amplified and the detection sensitivity can be enhanced. In the case of the following example, the secondary antibody is an antibody that specifically recognizes the anti-SP-D monoclonal antibody. When a secondary antibody is used, the procedure (1) to (5) described below may be used.
(1) The sample is added to the solid phase containing the immobilized anti-SP-D monoclonal antibody, and following an incubation period, the sample is removed and washed.
(2) The anti-SP-D monoclonal antibody is added, and incubation and washing are performed.
(3) An enzyme-labeled secondary antibody is added and incubated.
(4) A substrate is added to induce color development.
(5) Using a plate reader or the like, the color is measured to determine the amount of SP-D.

In those cases where the ELISA method is used as the method for measuring SP-D according to the present invention, the immobilized antibody that binds to the SP-D and the labeled antibody are of the same type, namely, are anti-SP-D monoclonal antibodies having the same amino acid sequence.

In the embodiments described above, the amount of SP-D can be quantified accurately regardless of the number of monomers constituting the SP-D multimer.

### <Measurement Kit>

An SP-D measurement kit according to one aspect of the present invention includes first reagent containing an insoluble carrier on which an anti-pulmonary surfactant protein D monoclonal antibody is immobilized, and a second reagent that contains a labeled antibody containing the anti-pulmonary surfactant protein D monoclonal antibody and a labeling material.

The insoluble carrier and the labeling material may be the same substance, and may be composed of latex particles. The SP-D monoclonal antibody immobilized on the insoluble carrier and the SP-D monoclonal antibody of the labeled antibody are of the same type, namely are antibodies having the same amino acid sequence.

The SP-D monoclonal antibody binds to a trimer of the pulmonary surfactant protein D, with only one monoclonal antibody binding to a single pulmonary surfactant protein D trimer. At least two SP-D monoclonal antibodies bind to dodecamers or 36-mers of the SP-D.

The SP-D monoclonal antibody, the insoluble carrier, the labeling material and the labeled antibody are as described above, and the content of those descriptions is applicable here.

The SP-D measurement kit according to one aspect of the present invention may also include at least one other test reagent or dilute specimen solution or the like such as standard antigen materials used in the measurement of SP-D and antigen samples used for accuracy control.

Other aspects described above such as the method for preparing the monoclonal antibody of the present invention, the nucleic acid molecule that encodes the monoclonal antibody of the present invention, the vector or plasmid containing that nucleic acid molecule, cells containing the nucleic acid molecule or vector or plasmid, and hybridomas for producing the antibody of the present invention are also included as subjects of the present invention.

### [Examples]

The present invention is described below in further detail using a series of examples, but the present invention is not limited to the following examples.

### <Preparation of Monoclonal Antibodies>

Recombinant human SP-D (hereinafter abbreviated as rSP-D) manufactured by GenScript Biotech Corporation was used as the immunogen. The rSP-D was used in a 1:1 mixture with Freund's Complete Adjuvant (manufactured by Difco Laboratories) for the initial immunization, and in a 1:1 mixture with Freund's Incomplete Adjuvant (manufactured by Difco Laboratories) for all subsequent immunizations. Balb/c mice or F344/Jcl rats were subjected to fortnightly subcutaneous immunizations using 25 µg of the immunogen for the initial immunization and 20 µg for the second and subsequent immunizations (diluted in PBS). Following the third immunization, the antibody titer in the blood was evaluated by antigen-immobilized ELISA. Individuals for which a satisfactory increase in titer was confirmed were subjected to intraperitoneal immunization with PBS-diluted immunogen one to three days prior to dissection. The spleen cells, iliac lymph node cells and inguinal lymph node cells were collected, and were fused with myeloma cells SP2/0 by electrical fusion. The fused cells (namely, the hybridoma) were cultivated in a 96-well plate, and 7 or 8 days after fusion, the culture supernatant was collected. Subsequently, screening was conducted using the antigen-immobilized ELISA method described below, and those lines exhibiting reactivity with rSP-D were selected. A medium exchange was conducted on the day prior to screening.

### < Screening of Anti-SP-D Antibodies >

An ELISA 96-well plate (NUNC 442404) was inoculated with rSP-D (1 µg/mL in PBS) in sufficient amount to achieve a concentration of 50 µL/well, and the plate was left to rest at room temperature for two hours. Following washing three times with 400 µL/well of PBST, a blocking solution (1% BSA-PBST) was dispensed at 100 µL/well, and the plate was then left to stand, either at room temperature for one hour or overnight at 4°C. Following removal of the blocking solution, the cell culture supernatant and a 1,000-fold or 10,000-fold diluted antiserum were each dispensed at 50 µL/well, and the plate was then left to stand at room temperature for one hour. Following washing three times with PBST, Goat anti-Mouse IgG (H+L) PAb-HRP (manufactured by SouthernBiotech Inc., 9,500-fold dilute solution) or Goat anti-Rat IgG (H+L) PAb-HRP (manufactured by SouthernBiotech Inc., 8,500-fold dilute solution) was dispensed at 50 µL/well,and the plate was left to stand at room temperature for one hour. Following washing three times with PBST, an OPD chromogenic solution was dispensed at 50 µL/well,and the plate was left to stand at room temperature for 10 minutes. A stop solution was then dispensed at 50 µL/well,and following stopping of the reaction, measurements were conducted using a plate reader (Absorbance: 492 nm). This enabled selection of antibodies that exhibited reactivity with the rSP-D. The selected anti-SP-D monoclonal antibodies are shown in Table 1.

**[Table 1]**

| Anti-SP-D antibody | IgG class |
|---|---|
| S21201R | IgG2ak |
| S21202 | IgG1k |
| S21203 | IgG1k |
| S21204 | IgG1k |
| S21205 | IgG1k |
| S21206 | IgG1k |
| S21207 | IgG1k |
| S21208 | IgG1k |

### <Analysis Example 1> Confirmation of Specificity of Anti-SP-D Monoclonal Antibodies

An ELISA 96-well plate (NUNC 442404) was inoculated with an antigen mixed solution containing rSP-D, recombinant human surfactant A (SP-A), recombinant human Collectin Liver 1 (CLL1), and recombinant human Mannan-Binding Lectin (MBL) (each component having a concentration of 1 µg/mL in PBS) at a rate of 50 µL/well, and the plate was then left to stand at room temperature for two hours. Following washing three times with 50 µL/well of PBST, a blocking solution (1% BSA-PBST) was dispensed at 100 µL/well,and the plate was then left to stand, either at room temperature for one hour or overnight at 4°C. Following removal of the blocking solution, each of the anti-SP-D monoclonal antibody solutions (5 µg/mL) shown in Table 2 was dispensed at 50 µL/well, and the plate was then left to stand at room temperature for one hour. Following washing three times with PBST, Goat anti-Mouse IgG (H+L) PAb-HRP (manufactured by SouthernBiotech Inc., 1031-05, 9,500-fold dilute solution) or Goat anti-Rat IgG (H+L) PAb-HRP (manufactured by SouthernBiotech Inc., 3050-15, 8,500-fold dilute solution) was dispensed at 50 µL/well,and the plate was left to stand at room temperature for one hour. Following washing three times with PBST, an OPD chromogenic solution was dispensed at 50 µL/well, and the plate was left to stand at room temperature for 10 minutes. A stop solution was then dispensed at 50 µL/well, and following stopping of the reaction, measurements were conducted using a plate reader (Absorbance: 492 nm). For each well, the result for the detected value, calculated by subtracting the measured value for a well into which no anti-SP-D monoclonal antibody had been dispensed from the measured value for each well, is shown in Table 2. All of the anti-SP-D monoclonal antibodies reacted strongly with only the rSP-D, and exhibited absolutely no reactivity or extremely little reactivity with the antigens other than SP-D.

**[Table 2]**

| | SP-D | SP-A | CLL1 | MBL |
|---|---|---|---|---|
| S21201R | 3.685 | -0.078 | 0.023 | 0.001 |
| S21202 | 1.197 | 0.005 | -0.001 | -0.002 |
| S21203 | 2.603 | 0.039 | 0.000 | -0.001 |
| S21204 | 2.351 | -0.002 | -0.001 | -0.001 |
| S21205 | 2.488 | -0.003 | 0.000 | 0.000 |
| S21206 | 2.508 | 0.000 | -0.001 | -0.001 |
| S21207 | 2.448 | 0.003 | 0.003 | 0.001 |
| S21208 | 2.026 | 0.011 | 0.014 | 0.008 |

### <Analysis Example 2> Evaluation of Reactivity Between Anti-SP-D Monoclonal Antibodies and Biological Sample-Derived SP-D

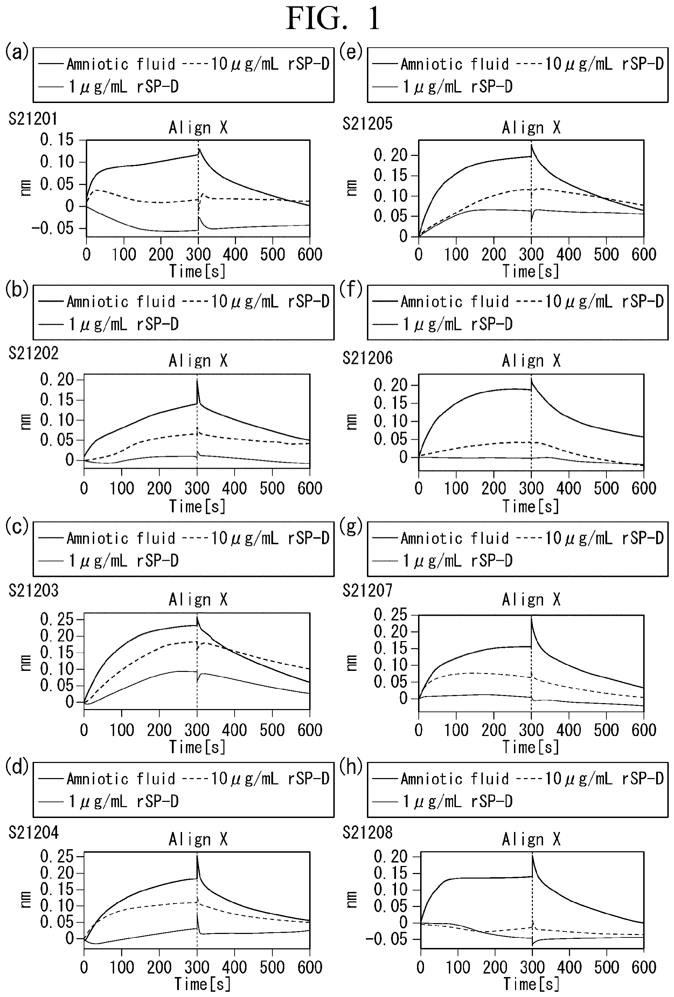

Using Octet (manufactured by Sartorius AG, Red 384), the reactivity between the anti-SP-D monoclonal antibodies and organism-derived SP-D contained in amniotic fluid was evaluated by measuring intermolecular interactions. A biotinylated anti-SP-D monoclonal antibody prepared by typical methods using a Biotin Labeling Kit-NH2 (manufactured by Dojindo Laboratories Co., Ltd.) was bound to the Streptavidin sensor to perform a blocking treatment. The reactivity between either amniotic fluid diluted 5-fold with a 1% BSA-containing PBST (phosphate buffer saline solution containing Tween (a registered trademark)) or rSP-D prepared at a concentration of 1 µg/mL or 10 µg/mL with each of the anti-SP-D monoclonal antibodies was then evaluated. The results are shown in FIG. 1. As illustrated in FIG. 1, all of the acquired anti-SP-D monoclonal antibodies reacted with rSP-D and the organism-derived SP-D contained in amniotic fluid.

### <Analysis Example 3> Detection of Biological Sample-Derived SP-D by Sandwich ELISA

An investigation was conducted to ascertain whether SP-D in blood serum could be detected using a combination of the acquired anti-SP-D monoclonal antibodies. Each well of an ELISA plate was inoculated with 50 µL/well of an anti-SP-D monoclonal antibody that had been prepared as an immobilized antibody at a concentration of 5 µg/mL in PBS, and the plate was then left to stand at room temperature for two hours. Each well was washed three times using 400 µL/well of TBST (washing solution) containing 5 mM of calcium chloride, a 1% BSA/TBST (blocking solution) was then dispensed into each well at 100 µL/well,and the plate was left to stand at room temperature for one hour. The blocking solution was then removed from each well, a solution prepared by diluting blood serum with a 5 mM calcium chloride-containing 1% BSA/TBST solution to achieve an SP-D concentration of 100 ng/mL was dispensed into each well at a rate of 50 µL/well,and the plate was then left to stand at room temperature for one hour. Each well was then washed three times using 400 µL/well of the washing solution, a solution prepared by diluting a biotinylated anti-SP-D monoclonal antibody, prepared by typical methods using a Biotin Labeling Kit-NH2 (manufactured by Dojindo Laboratories Co., Ltd.), to a concentration of 0.2 µg/mL with a 5 mM calcium chloride-containing 1% BSA/TBST was dispensed at 50 µL/well, and the plate was then left to stand at room temperature for one hour. Following washing of each well three times using 400 µL/well of the washing solution, an HRP-Streptavidin solution prepared at a concentration of 0.2 µg/mL in TBS containing 5 mM of calcium chloride was dispensed at 50 µL/well, and the plate was left to stand at room temperature for 30 minutes. Following washing three times with the washing solution, an OPD chromogenic solution was dispensed at 50 µL/well, and the plate was left to stand at room temperature for 10 minutes. A stop solution was then dispensed at 50 µL/well,and absorbance measurements were conducted at a wavelength of 492 nm using a plate reader.

The results are shown in Table 3, with antibody combinations for which the absorbance was less than 0.1 recorded as -, monoclonal antibody combinations for which the absorbance was at least 0.1 but less than 0.5 recorded as +, monoclonal antibody combinations for which the absorbance was at least 0.5 but less than 1.0 recorded as ++, and monoclonal antibody combinations for which the absorbance was 1.0 or higher recorded as +++. A plurality of antibody combinations including combinations of the same monoclonal antibody were able to detect the SP-D in blood serum. In particular, combinations of the S21202, S21205 and S21208 antibodies were able to detect the biological sample-derived SP-D with high sensitivity.

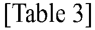

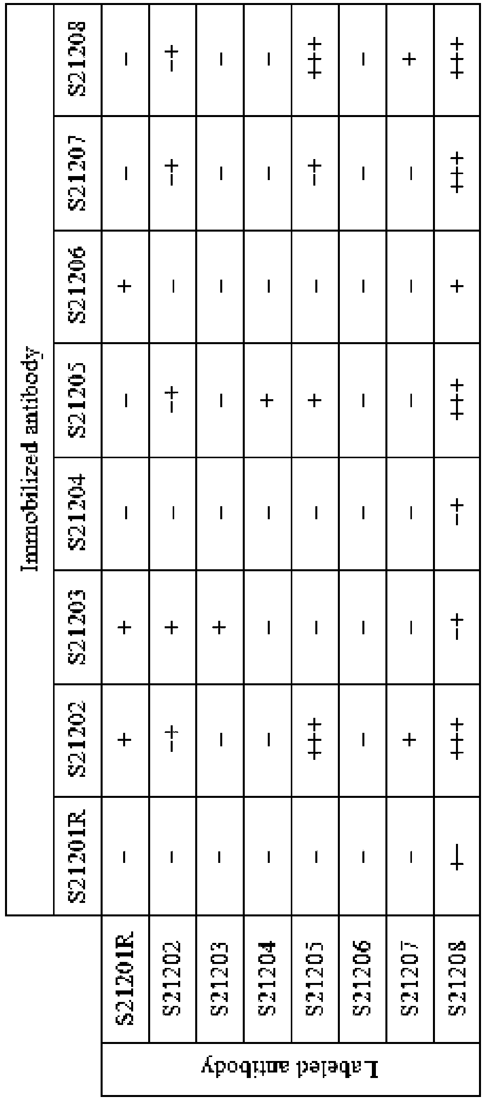

### <Analysis Example 4> rSP-D Multimer Distribution Analysis 1

A solution containing 55 µg of rSP-D manufactured by GenScript Biotech Corporation was added to a Superdex 200 Increase 10/300 GL column (Cytiva Inc.) equilibrated with TBS, and gel filtration chromatography was conducted at a flow rate of 0.7 mL/minute. The UV absorbance was recorded during the chromatography. Eluate samples of 400 µL were collected in tubes containing 100 µL of 5% BSA-containing TBA. Following collection, each fraction was mixed thoroughly and then dispensed and stored at -30°C.

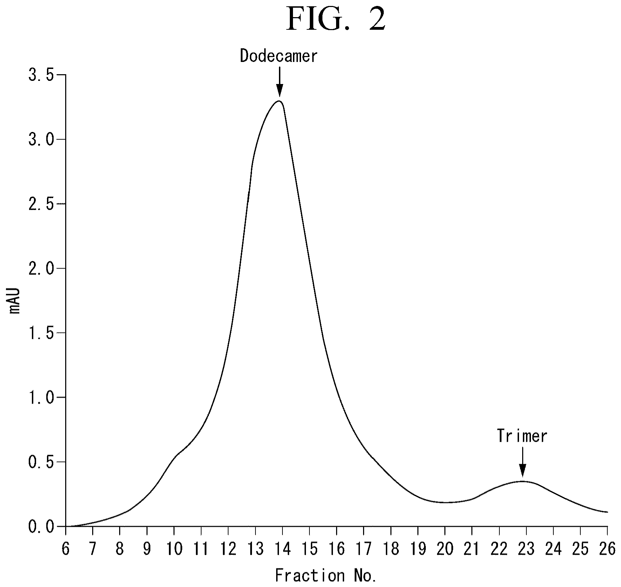

FIG. 2 shows the chromatogram illustrating the results of analyzing the rSP-D manufactured by GenScript Biotech Corporation by gel filtration chromatography. A dodecamer of SP-D was eluted mainly in fraction 14, and a trimer of SP-D was eluted mainly in fraction 23.

### <Detection 1 of rSP-D Gel Filtration Fractions by Anti-SP-D Monoclonal Antibodies>

Using the anti-SP-D monoclonal antibodies S21208, S21205 and S21202, four ELISA systems were used, namely S21208 solid phase - S21208 liquid phase (Example 1), S21202 solid phase - S21202 liquid phase (Example 2), S21205 solid phase - S21208 liquid phase (Comparative Example 1) and S21202 solid phase - S21205 liquid phase (Comparative Example 2), and each fraction obtained in Analysis Example 4 was measured for rSP-D using the procedure described below.

An ELISA plate was inoculated with 50 µL/well of the immobilized antibody prepared at a concentration of 5 µg/mL in PBS, and the plate was left to stand at room temperature for two hours. Following washing three times using 400 µL/well of TBST containing 5 mM of calcium chloride, a 1% BSA/TBST (blocking solution) was dispensed into each well at 100 µL/well, and the plate was left to stand at room temperature for one hour. Following removal of the blocking solution, each of the fractions obtained in Analysis Example 4 diluted 1,000-fold with 1% BSA/TBST containing 5 mM of calcium chloride was dispensed at 50 µL/well,and the plate was then left to stand at room temperature for one hour. Following washing three times using 400 µL/well of the washing solution, a solution prepared by diluting a biotinylated antibody prepared by typical methods using a Biotin Labeling Kit-NH2 (manufactured by Dojindo Laboratories Co., Ltd.) to a concentration of 0.2 µg/mL with a 5 mM calcium chloride-containing 1% BSA/TBST was dispensed at 50 µL/well, and the plate was then left to stand at room temperature for one hour. Following washing of each well three times using 400 µL/well of the washing solution, an HRP-Streptavidin solution prepared at a concentration of 0.2 µg/mL in TBS containing 5 mM of calcium chloride was dispensed at 50 µL/well, and the plate was left to stand at room temperature for 30 minutes. Following washing three times with the washing solution at 400 µL/well, an OPD chromogenic solution was dispensed at 50 µL/well,and the plate was left to stand at room temperature for 10 minutes. A stop solution was then dispensed at 50 µL/well, and absorbance measurements were conducted at a wavelength of 492 nm using a plate reader.

The results are shown in Table 4. In Examples 1 and 2 in which sandwich detection of the SP-D was conducted using the same anti-SP-D monoclonal antibody, the dodecamer of SP-D in fraction 14 was detected, but the trimer of SP-D in fraction 23 was not detected. In Comparative Examples 1 and 2 in which sandwich detection of the SP-D was conducted using two different types of anti-SP-D monoclonal antibodies, both the dodecamer and the trimer of SP-D were able to be detected. Based on these results, it was evident that by conducting sandwich detection of SP-D using the same anti-SP-D monoclonal antibody, it was possible to detect only multimers of SP-D larger than the trimer.

**[Table 4]**

| | | Example 1 | Example 2 | Comparative Example 1 | Comparative Example 2 |
|---|---|---|---|---|---|
| Fraction | SP-D | S21208 solid phase - S21208 liquid phase | S21202 solid phase - S21202 liquid phase | S21205 solid phase - S21208 liquid phase | S21202 solid phase - S21205 liquid phase |
| Fraction 14 | dodecamer | 0.652 | 0.278 | 1.557 | 2.617 |
| Fraction 23 | trimer | 0.006 | 0.003 | 0.381 | 0.137 |

### <Analysis Example 5> rSP-D Multimer Distribution Analysis 2

Using the same method as Analysis Example 4, rSP-D manufactured by either R&D Systems, Inc. or GenScript Biotech Corporation was subjected to gel filtration chromatography. In each case, 217 µg of the rSP-D was subjected to gel filtration chromatography.

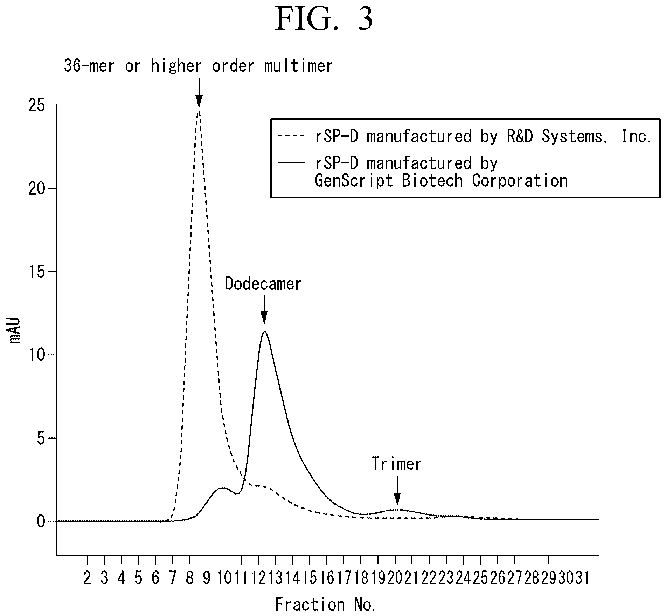

FIG. 3 shows the chromatogram illustrating the results of analyzing the rSP-D manufactured by R&D Systems, Inc., and the rSP-D manufactured by GenScript Biotech Corporation using gel filtration chromatography. The SP-D 36-mer and higher order multimers were eluted mainly in fraction 8, the dodecamer of SP-D was eluted mainly in fraction 12, and the trimer of SP-D was eluted mainly in fraction 20. In the rSP-D from R&D Systems, Inc., the 36-mer and higher order multimers of SP-D represented the main structure, whereas in the rSP-D from GenScript Biotech Corporation, the dodecamer of SP-D represented the main structure. In the following description, the nth fraction eluted during the analysis of the SP-D from R&D Systems, Inc. is termed Rn, and the nth fraction eluted during the analysis of the SP-D from GenScript Biotech Corporation is termed Gn. The UV absorbance of the fraction R8 containing a large amount of the SP-D 36-mer and higher order multimers was 24.68 mAU, and the UV absorbance of the fraction G12 containing a larger amount of the dodecamer of SP-D was 11.53 mAU. Because the rSP-D protein concentration is proportional to the UV absorbance, the protein ratio between the SP-D 36-mer and higher order multimers in fraction R8 and the SP-D dodecamer in fraction G12 was 2.14:1.

### <Detection 2 of rSP-D Gel Filtration Fractions>

Using the anti-SP-D monoclonal antibodies S21208, S21205 and S21202, three ELISA systems were used, namely S21208 solid phase - S21208 liquid phase (Example 1), S21205 solid phase - S21208 liquid phase (Comparative Example 1) and S21202 solid phase - S21205 liquid phase (Comparative Example 2), and the rSP-D in each fraction obtained in Analysis Example 5 was measured using the same procedure as that described above in <Detection 1 of rSP-D Gel Filtration Fractions by Anti-SP-D Monoclonal Antibodies>.

The results are shown in Table 5. In Example 1 in which sandwich detection of the SP-D was conducted using the same anti-SP-D monoclonal antibody, the ratio between the absorbance measured for the fraction R8 containing the SP-D 36-mer and higher order multimers and the absorbance measured for the fraction G12 containing the SP-D dodecamer was 2.76:1, which was similar to the protein ratio of 2.14:1 between the SP-D 36-mer and higher order multimers and the SP-D dodecamer in the gel filtration chromatography. Further, in Example 1, the SP-D trimer contained in fraction G20 was not detected.

In Comparative Examples 1 and 2 in which sandwich detection of the SP-D was conducted using two different types of anti-SP-D monoclonal antibodies, detection of the SP-D trimer was possible. On the other hand, the ratio between the absorbance measured for the fraction R8 containing the SP-D 36-mer and higher order multimers and the absorbance measured for the fraction G12 containing the SP-D dodecamer was 1:0.87 and 1:0.44 respectively, which does not reflect the protein ratio of between the various multimers observed in the gel filtration chromatography. More specifically, the absorbance measured for the fraction containing the SP-D dodecamer, which based on the results of the gel filtration chromatography should exhibit an amount of protein lower than that of the fraction containing the SP-D 36-mer and higher order multimers, was actually higher than the absorbance measured for the fraction containing the SP-D 36-mer and higher order multimers.

Based on these results, it was thought that by conducting sandwich detection of the SP-D dodecamer and higher order multimers using the same anti-SP-D monoclonal antibody, it was possible to obtain measurements that reflected the actual amount of protein, regardless of the number of basic units in the SP-D multimer.

**[Table 5]**

| | | Example 1 | Comparative Example 1 | Comparative Example 2 |
|---|---|---|---|---|
| Fraction | SP-D | S21208 solid phase - S21208 liquid phase | S21205 solid phase - S21208 liquid phase | S21202 solid phase - S21205 liquid phase |
| Fraction R8 | 36-mer and higher | 0.746 | 0.446 | 0.313 |
| Fraction G12 | dodecamer | 0.27 | 0.512 | 0.704 |
| Fraction G20 | trimer | -0.007 | 0.078 | 0.022 |
| | | | | |
| Absorbance for 36-mer and higher / absorbance for dodecamer | | 2.76 | 0.87 | 0.44 |

### <Preparation of LTIA Reagent for SP-D Measurement>

### 1) First Reagent

A solution of the following composition was prepared as the first reagent.
100 mM MES-NaOH (pH 6.0)
500 mM NaCl
0.5% BSA

### 2) Anti-Human SP-D Monoclonal Antibody-Sensitized Latex Particle Solution

To a 1% polystyrene latex solution with an average particle size of 317 nm and a critical aggregation concentration of 280 mM (manufactured by Sekisui Medical Co., Ltd.) (10 mM MOPS buffer solution) was added an equal volume of an anti-SP-D antibody solution that had been diluted to 0.35 mg/mL with 10 mM MOPS buffer solution, and the resulting mixture was stirred at 4°C for two hours. Subsequently, an equal volume of 10 mM MOPS buffer solution containing 0.5% BSA was added, and stirring was continued at 4°C for one hour, thus producing an anti-human SP-D monoclonal antibody-sensitized latex solution.

### 3) Second Reagent

### 3-1) Example 2

A second reagent was prepared by diluting the S21208 antibody-sensitized latex particle solution prepared using the procedure described above in 2) with sufficient 5 mM MOPS-NaOH (pH 7.0) buffer to achieve an absorbance at a wavelength of 600 nm of 6.0 Abs.

### 3-2) Comparative Example 3

The S21205 antibody-sensitized latex particle solution and the S21208 antibody-sensitized latex particle solution prepared using the procedure described above in 2) were each diluted with sufficient 5 mM MOPS-NaOH (pH 7.0) buffer to achieve absorbances at a wavelength of 600 nm of 3.0 Abs and 6.0 Abs. respectively, and the two solutions were then mixed together to form a second reagent.

### <Quantification of rSP-D Gel Filtration Fractions using SP-D Measurement Reagents>

### 1) Measurement Method using LTIA Reagent

The first reagent and the second reagent were combined, and using a biochemical autoanalyzer (Hitachi Automatic Analyzer 3500, manufactured by Hitachi, Ltd.), the procedure described below was used to measure and calculate the SP-D concentration in each of the fractions obtained in Analysis Example 5 (Example 2 and Comparative Example 3). First, 120 µL of the first reagent was added to 5 µL of each of the fractions, and each mixture was heated at 37°C for 5 minutes. Next, 40 µL of the second reagent was added to each fraction and stirred. The change in absorbance across a 5-minute period was measured at a principal wavelength of 570 nm and a secondary wavelength of 800 nm. The concentration of SP-D in each fraction was calculated by using a calibration curve produced by plotting the change in absorbance per unit of time upon measurement of recombinant SP-D solutions (manufactured by R&D Systems, Inc.) of known concentration along the horizontal axis, and the concentration of the recombinant SP-D along the vertical axis.

### 2) Measurement Method using CL SP-D Yamasa NX (a certified in vitro diagnostic reagent manufactured by Yamasa Corporation (hereinafter referred to as "the Certified Reagent"))

Each of the fractions obtained in Analysis Example 5 was measured using the certified reagent in accordance with the package insert (Comparative Example 4). According to the package insert of the certified reagent, this regent is used for measuring SP-D in blood serum using a chemiluminescent enzyme immunoassay method that utilizes two different types of anti-human SP-D mouse monoclonal antibodies.

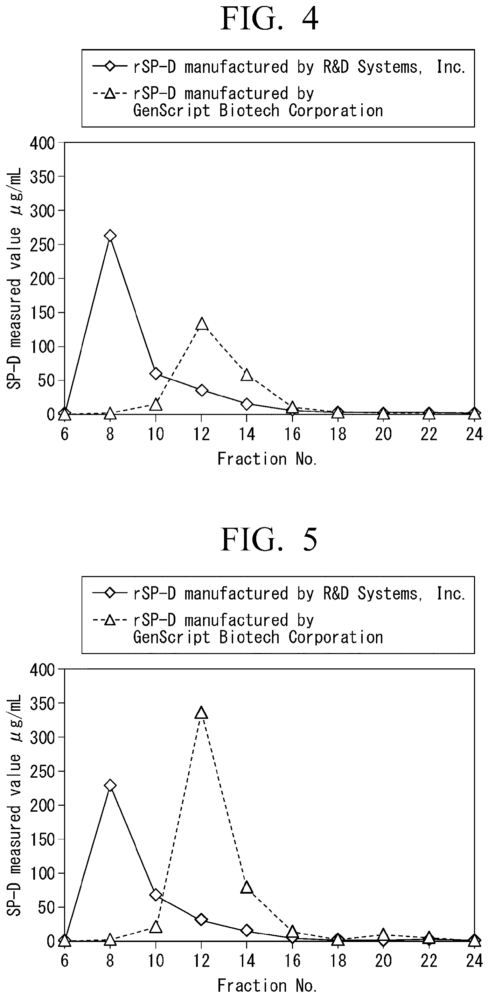

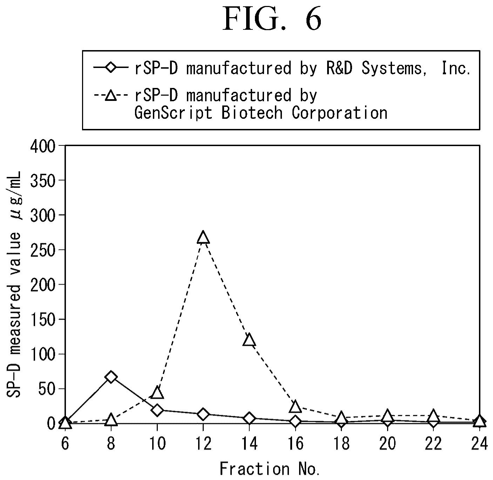

Table 6 shows the results of measuring the SP-D concentration for the fractions R8, R12 and R20 obtained in Analysis Example 5. FIG. 4 is a graph illustrating the measured value for the SP-D concentration in each of the fractions obtained in Analysis 5 in Example 2. FIG. 5 is a graph illustrating the measured value for the SP-D concentration in each of the fractions obtained in Analysis 5 in Comparative Example 3. FIG. 6 is a graph illustrating the measured value for the SP-D concentration in each of the fractions obtained in Analysis 5 in Comparative Example 4.

In Example 2, in a similar manner to that observed for the ELISA in Example 1, SP-D was detected with a similar mass ratio to that observed for the SP-D protein mass ratio (2.14:1) between the 36-mer and higher order multimers and the dodecamer in the gel filtration chromatography. Further, in Example 2, the measured value for the fraction G20 containing the SP-D trimer, and the measured value for the fraction G18 which exhibited a lower measured protein mass value than the fraction G20 in the gel filtration chromatography were similar, indicating that the trimer of SP-D was substantially undetected in Example 2.

On the other hand, in Comparative Example 3 and Comparative Example 4 which used two different types of antibodies, the results differed from those of the gel filtration chromatography, with the measured value for the fraction G12 containing the dodecamer of SP-D being calculated as lower than that of the measured value for the fraction R8 containing the 36-mer and higher order multimers. Further, in both Comparative Example 3 and Comparative Example 4, the measured value for the fraction G20 containing the trimer of SP-D was higher than the measured value for the fraction G18, indicating that the SP-D trimer was being detected (see FIGS. 5 and 6). Based on the above results, it was evident that when sandwich detection of SP-D was conducted using the same antibody, it was possible to obtain measurements that reflected the actual amount of protein even using the LTIA reagent, regardless of the number of basic units in the SP-D multimer.

**[Table 6]**

| | | Example 2 | Comparative Example 3 | Comparative Example 4 |
|---|---|---|---|---|
| Fraction | SP-D | S21208 antibody LTIA reagent | S21205 antibody S21208 antibody LTIA reagent | Certified reagent |
| Fraction R8 | 36-mer and higher | 263 | 230 | 66 |
| Fraction G12 | dodecamer | 134 | 337 | 268 |
| Fraction G20 | trimer | 0.8 | 8.7 | 10 |
| | | | | |
| Absorbance for 36-mer and higher / absorbance for dodecamer | | 1.97 | 0.68 | 0.24 |

### [Industrial Applicability]

The aspects described above are able to provide a measurement method and measurement kit for pulmonary surfactant protein D that enable accurate quantification regardless of the number of monomers constituting the pulmonary surfactant protein D multimer, and also to provide a monoclonal antibody that can be used in this measurement method and measurement kit, and a cell that produces this antibody.

### [Accession Numbers]

NPMD NITE BP-03825
NPMD NITE BP-03826
NPMD is an acronym for the National Institute of Technology and Evaluation Patent Microorganisms Depositary (Room 122, 2-5-8 Kazusakamatari, Kisarazu-shi, Chiba, Japan).

## Claims

1. A method for measuring pulmonary surfactant protein D, the method comprising:
a step of bringing a sample containing pulmonary surfactant protein D into contact with an insoluble carrier on which an anti-pulmonary surfactant protein D monoclonal antibody has been immobilized, and
a step of detecting a complex of the pulmonary surfactant protein D and at least two of the anti-pulmonary surfactant protein D monoclonal antibodies, wherein
only one type of antibody is immobilized on the insoluble carrier as the anti-pulmonary surfactant protein D monoclonal antibody.

2. The method according to Claim 1, wherein the insoluble carrier is composed of latex particles.

3. The method according to Claim 1, wherein
the insoluble carrier has a plate-like form, and
the complex contains the anti-pulmonary surfactant protein D monoclonal antibody immobilized on the insoluble carrier, and a labeled antibody comprising the anti-pulmonary surfactant protein D monoclonal antibody and a labeling material.

4. The method according to Claim 1, wherein the sample is blood serum or blood plasma.

5. The method according to any one of Claims 1 to 4, wherein
the pulmonary surfactant protein D comprises at least one basic unit composed of a trimer of pulmonary surfactant protein D, and
only one anti-pulmonary surfactant protein D monoclonal antibody binds to a single basic unit.

6. The method according to Claim 5, wherein
in the step of detecting the complex, trimers of pulmonary surfactant protein D are not detected.

7. The method according to Claim 6, wherein
in the step of detecting the complex, pulmonary surfactant protein D having two or more assembled basic units is detected.

8. A measurement kit for pulmonary surfactant protein D comprising:
a first reagent containing an insoluble carrier on which an anti-pulmonary surfactant protein D monoclonal antibody has been immobilized, and
a second reagent containing a labeled antibody comprising the anti-pulmonary surfactant protein D monoclonal antibody and a labeling material.

9. The measurement kit according to Claim 8, wherein the insoluble carrier and the labeling material are the same substance.

10. A monoclonal antibody that binds to a trimer of pulmonary surfactant protein D, wherein
only one antibody binds to a single trimer of pulmonary surfactant protein D.

11. The monoclonal antibody according to Claim 10, wherein at least two antibodies bind to a dodecamer or higher order multimer of pulmonary surfactant protein D.

12. A hybridoma that produces the monoclonal antibody according to Claim 10 or 11.

13. A hybridoma deposited with National Institute of Technology and Evaluation Patent Microorganisms Depositary with an accession number NITE BP-03825 or an accession number NITE BP-03826.
